# FASCICULE DE BREVET EUROPEEN

(11) **EP 1 878 470 B1**
(45) Date de publication et mention de la délivrance du brevet: **11.03.2009**
(21) Numéro de dépôt: 07105023.1
(22) Date de dépôt: 27.03.2007
(51) Int. Cl.: A61Q 19/02, A61K 8/49

(54) **Procédé de dépigmentation de la peau**
Verfahren zur Depigmentierung der Haut
Skin depigmentation method

(30) Priorité: 28.04.2006 FR 0651520
(43) Date de publication de la demande: 16.01.2008
(73) Titulaire: L'Oréal, 75008 Paris (FR)
(72) Inventeur: DALKO, Maria, 91190, GIF S/YVETTE (FR)
(74) Mandataire: Kromer, Christophe

(56) Documents cités:
- EP-A1- 1 547 577
- WO-A-98/15276
- DE-A1- 2 034 784
- DE-A1- 10 132 338

## Description

La présente invention concerne un procédé cosmétique pour dépigmenter et/ou blanchir la peau utilisant un composé dérivé d'adénosine.

La couleur de la peau humaine est fonction de différents facteurs et notamment des saisons de l'année, de la race et du sexe; elle est principalement déterminée par la nature et la concentration de mélanine produite par les mélanocytes. Les mélanocytes sont les cellules spécialisées qui, par l'intermédiaire d'organelles particuliers, les mélanosomes, synthétisent la mélanine. En outre, à différentes périodes de leur vie, certaines personnes voient apparaître sur la peau et plus spécialement sur les mains, des taches plus foncées et/ou plus colorées, conférant à la peau une hétérogénéité. Ces taches sont dues aussi à une concentration importante de mélanine dans les kératinocytes situés à la surface de la peau.

L'utilisation de substances dépigmentantes topiques inoffensives présentant une bonne efficacité est tout particulièrement recherchée en vue de traiter les hyperpigmentations régionales par hyperactivité mélanocytaire telles que les mélasmas idiopathiques, survenant lors de la grossesse ("masque de grossesse" ou chloasma) ou d'une contraception oestro-progestative, les hyperpigmentations localisées par hyperactivité et prolifération mélanocytaire bénigne, telles que les taches pigmentaires séniles dites lentigo actiniques, les hyperpigmentations accidentelles, éventuellement dues à la photosensibilisation ou à la cicatrisation post-lésionnelle, ainsi que certaines leucodermies, telles que le vitiligo. Pour ces dernières (les cicatrisations pouvant aboutir à une cicatrice donnant à la peau un aspect plus blanc), à défaut de pouvoir repigmenter la peau lésée, on achève de dépigmenter les zones de peau normale résiduelle pour donner à l'ensemble de la peau une teinte blanche homogène.

Le mécanisme de formation de la pigmentation de la peau, c'est-à-dire de la formation de la mélanine est particulièrement complexe et fait intervenir schématiquement les principales étapes suivantes :

**Tyrosine ---> Dopa ---> Dopaquinone ---> Dopachrome ---> Mélanine**

La tyrosinase (monophénol dihydroxyl phénylalanine : oxygen oxydo-reductase EC 1.14.18.1) est l'enzyme essentielle intervenant dans cette suite de réactions. Elle catalyse notamment la réaction de transformation de la tyrosine en Dopa (dihydroxyphénylalanine) grâce à son activité hydroxylase et la réaction de transformation de la Dopa en dopaquinone grâce à son activité oxydase. Cette tyrosinase n'agit que lorsqu'elle est à l'état de maturation sous l'action de certains facteurs biologiques.

Une substance est reconnue comme dépigmentante si elle agit directement sur la vitalité des mélanocytes épidermiques où se déroule la mélanogénèse, et/ou si elle interfère avec une des étapes de la biosynthèse de la mélanine soit en inhibant une des enzymes impliquées dans la mélanogénèse, soit en s'intercalant comme analogue structural d'un des composés chimiques de la chaîne de synthèse de la mélanine, chaîne qui peut alors être bloquée et ainsi assurer la dépigmentation.

Les substances les plus utilisées en tant que dépigmentants sont plus particulièrement l'hydroquinone et ses dérivés, en particulier ses éthers tels que le monométhyléther et le monoéthyléther d'hydroquinone. Ces composés, bien qu'ils présentent une efficacité certaine, ne sont malheureusement pas exempts d'effets secondaires du fait de leur toxicité, ce qui peut rendre leur emploi délicat, voire dangereux. Cette toxicité provient de ce qu'ils interviennent sur des mécanismes fondamentaux de la mélanogénèse en tuant des cellules qui risquent alors de perturber leur environnement biologique et qui par conséquent obligent la peau à les évacuer en produisant des toxines.

Ainsi, l'hydroquinone est un composé particulièrement irritant et cytotoxique pour le mélanocyte, dont le remplacement, total ou partiel a été envisagé par de nombreux auteurs.

On a ainsi cherché des substances qui n'interviennent pas dans le mécanisme de la mélanogénèse mais qui agissent en amont sur la tyrosinase en empêchant son activation et sont de ce fait beaucoup moins toxiques. On utilise couramment comme inhibiteur de l'activation de la tyrosinase l'acide kojique qui complexe le cuivre présent dans le site actif de cette enzyme. Malheureusement, ce composé est instable en solution, ce qui complique quelque peu la fabrication de la composition.

Il subsiste le besoin d'un nouvel agent blanchissant de la peau humaine à action aussi efficace que ceux connus, mais n'ayant pas leurs inconvénients, c'est-à-dire qui soit non irritant, non toxique et/ou non allergisant pour la peau, tout en étant stable dans une composition, ou bien alternativement qui possède une action renforcée de façon à pouvoir être utilisé en quantité plus faible, ce qui diminue considérablement les effets secondaires observés.

A cet égard la Demanderesse a de manière surprenante et inattendue découvert que certains composés d'adénosine présentaient une bonne activité dépigmentante, même à faible concentration, sans faire preuve de cytotoxicité.

De façon plus précise, l'invention a donc pour objet un procédé cosmétique de dépigmentation et/ou de blanchiment de la peau humaine comprenant l'application sur la peau d'une composition cosmétique comprenant, dans un milieu physiologiquement acceptable, au moins un composé de formule (I) suivante : 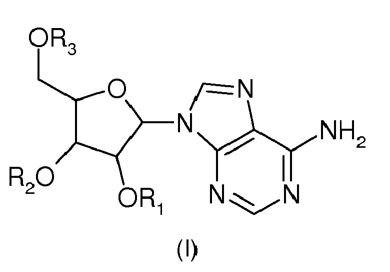 dans laquelle :
- R₁ et R₂ identiques désignent un radical hydrocarboné linéaire saturé en C₁-C₆ ou insaturé en C₂-C₆ , ou ramifié en C₃-C₆ saturé ou insaturé, ou bien encore forment ensemble, avec les atomes d'oxygène auxquels ils sont rattachés un radical isopropylidène ;
- R₃ désigne :
   (i) un radical hydrocarboné linéaire saturé en C₁-C₁₀ ou insaturé en C₂-C₁₀ , ou ramifié en C₃-C₁₀ saturé ou insaturé, éventuellement substitué par au moins un groupement choisi parmi -OR', -NR'R", -COOR', -CONR'R", -CF₃, -F , -OCF₃, -CN, -NO₂,
      ou (ii) un groupe -COR₄ avec R₄ désignant un radical hydrocarboné linéaire saturé en C₁-C₉ ou insaturé en C₂-C₉ , ou ramifié en C₃-C₉ saturé ou insaturé, éventuellement substitué par au moins un groupement choisi parmi -OR', -NR'R", -COOR', -CONR'R", - CF3, -F , -OCF₃, -CN, -NO₂ ;
      ou (iii) un groupe ester issu de la biotine ;
R' et R" désignant un atome d'hydrogène, un radical hydrocarboné linéaire saturé en C₁-C₆ ou insaturé en C₂-C₆ , ou ramifié en C₃-C₆ saturé ou insaturé, éventuellement substitué par au moins un groupement choisi parmi -OZ, -NZZ', -COOZ, Z et Z' désignant, indépendamment l'un de l'autre, un atome d'hydrogène ou un radical hydrocarboné linéaire saturé en C₁-C₆ ou insaturé en C₂-C₆, ou ramifié en C₃-C₆ saturé ou insaturé ;
et ses sels, isomères optiques et solvates,

Les composés de formule (I) selon l'invention permettent de dépigmenter et/ou d'éclaircir efficacement la peau d'êtres humains. Ils sont notamment destinés à être appliqués sur la peau d'individus présentant des taches de pigmentation brunâtres, des taches de sénescence, ou sur la peau d'individus désirant combattre l'apparition d'une couleur brunâtre provenant de la mélanogénèse, par exemple à la suite d'une exposition aux rayonnements ultra-violet.

Le procédé convient notamment pour éliminer les taches pigmentaires brunâtres et/ou les taches de sénescence, et/ou pour éclaircir la peau brunie.

L'invention a encore pour objet l'utilisation cosmétique d'un composé de formule (I) tel que décrit précédemment comme agent blanchissant et/ou dépigmentant de la peau, notamment pour éliminer les taches pigmentaires, les taches de sénescence et/ou en tant qu'agents anti-brunissement.

L'invention a aussi pour objet l'utilisation d'un composé de formule (I) tel décrit précédemment pour la fabrication d'une composition dermatologique destinée à dépigmenter et/ou blanchir la peau.

Certains des composés de formule (I) sont connus de l'art antérieur et décrits dans les documents suivants :
- WO-A-2004/037159 décrit le 2', 3'-isopropylidène- 5'-acétyl-adénosine (composé 265 page 203) dans une composition pharmaceutique pour le traitement de l'obésité. Ce document ne décrit pas d'appliquer la composition par voie topique sur la peau pour dépigmenter cette dernière ;
- Poppe, L et al ; "Synthesis and characterization of (5'-deoxyadenosin-5'-yl)cobalamin (='adenosylcobalamin') analogs mimicking the transition-state geometry of coenzyme-B12-dependent rearrangements" ; Helvetica Chimica Acta (1993), 76(6), 2367-83 ;
- Jones, A. S. et al ; "Synthetic analogs of polynucleotides. VII. Syntheses of 5'-O-acryloylnucleosides and copolymers of these with other acryloyl compounds" ; Journal of the Chemical Society [Section] C: Organic (1971), (19), 3183-7 ;
- Mornet, D. et al ; "The reaction of myosin with a bromoalkyl analog of adenosine triphosphate" ; FEBS Letters (1977), 84(2), 362-6 ;
- Huber Gerhard ; "esters of adenosine with organic and inorganic" acids; Chem. Ber. 89, 2853-62 (1956) - ref CA52:2027g
- Takemoto, K. et al; "Nucleic acid analogs: their specific interaction and applicability" ; Polymeric Materials Science and Engineering (1988), 58, 250-3 ;
- Purkayastha, Bhupesh C.; Bhattacharyya, S. N ; "Use of Ca oxalate monohydrate in the investigation of rare earth and thorium activities" ; J. Indian. Chem. Soc. 34, 427-33 (1957) - ref CA52:2627h ;
- Peterli, Stefan et al ; "Nitrostyrene derivatives of adenosine 5'-glutarates as selective inhibitors of the epidermal growth factor receptor protein tyrosine kinase" ; Helvetica Chimica Acta (1992). 75(3), 696-706.

Le terme alkyle, dans le cadre de la présente invention, signifie une chaîne hydrocarbonée saturée ou insaturée. Parmi les groupes alkyle convenant à la mise en oeuvre de l'invention, on peut notamment citer les groupes méthyle, éthyle, isopropyle, n-propyle, n-butyle, t-butyle, isobutyle, sec-butyle , pentyle, n-hexyle, cyclopropyle, cyclopentyle, cyclohexyle, allyle.

Comme sels des composé de formule (I), on peut citer les sels obtenus par addition de composé de formule (I) avec un acide minéral, choisi notamment parmi les acides chlorhydrique, sulfurique et phosphorique, ou avec un acide organique, choisi en particulier parmi les acides acétique, propionique, succinique, fumarique, lactique, glycolique, citrique et tartrique.

De préférence, les sels des composés (I) sont choisis parmi les sels obtenus à partir de l'acide chlorhydrique ou l'acide acétique ou l'acide citrique.

Dans la formule (I), les groupes hydrocarbonés (ou alkyle) peuvent notamment être choisis, selon le cas, parmi les groupes : méthyle, éthyle, n-propyle, isopropyle, n-butyle, isobutyle, tert-butyle, pentyle, hexyle, heptyle, octyle, nonyle, décyle.

Des radicaux R₁ et R₂ préférés décrits précédemment sont ceux formant ensemble, avec les atomes d'oxygène auxquels ils sont rattachés, un radical isopropylidène.

Pour les composés de formule (I), on préfère ceux ayant les significations suivantes :
- R₁ et R₂ forment ensemble, avec les atomes d'oxygène auxquels ils sont rattachés, un radical isopropylidène ;
- R₃ désigne :
   (i) un radical hydrocarboné linéaire saturé en C₁-C₁₀ ou insaturé en C₂-C₁₀ , ou ramifié en C₃-C₁₀, saturé ou insaturé ;
ou (ii) un groupe -COR₄ avec R₄ désignant un radical hydrocarboné linéaire en C₁-C₉ ou ramifié en C₃-C₉, saturé ou insaturé ;
ou (iii) un groupe ester issu de la biotine.

Préférentiellement, on utilise des composés de formule (I) pour lesquels :
- R₁ et R₂ forment ensemble, avec les atomes d'oxygène auxquels ils sont rattachés, un radical isopropylidène ;
- R₃ désigne :
   (i) un radical hydrocarboné linéaire en C₁-C₁₀ ;
ou (ii) un groupe -COR₄ avec R₄ désignant un radical hydrocarboné linéaire saturé en C₁-C₉ ;
ou (iii) un groupe ester issu de la biotine.

### Plus préférentiellement, on utilise des composés de formule (I) pour lesquels :

R₁ et R₂ forment ensemble, avec les atomes d'oxygène auxquels ils sont rattachés, un radical isopropylidène ;
R₃ désigne :
- un groupe -COR₄ avec R₄ désignant un radical hydrocarboné linéaire en C₁-C₉ ;
- ou un groupe ester issu de la biotine.

Les composés (I) peuvent être préparés selon l'un des quatre procédé de synthèse décrits ci-après en fonction de la signification des radicaux R₁ , R₂ et R₃ .

### Premier procédé

Les composés de formule (I) pour lesquels R₁ = R₂ et R₃ désignent un radical hydrocarboné tels que définis précédemment, peuvent notamment être préparés selon le schéma I réactionnel suivant :

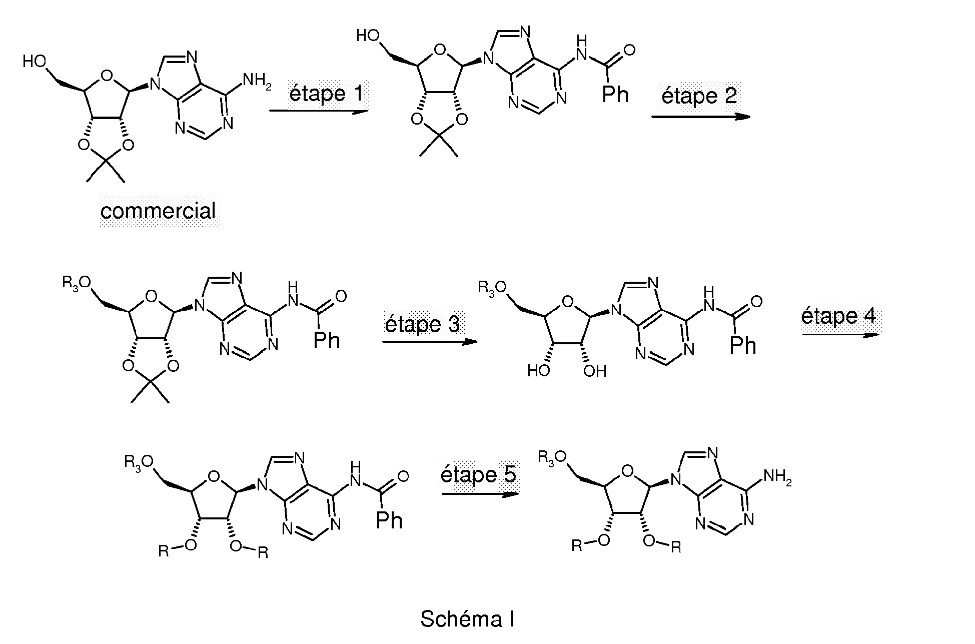

Une telle méthode de préparation est notamment décrite dans Helvetica Chimica Acta, 1993, (vol 76), page 2367.

Selon l'étape 1, on fait réagir l'isopropylidène adénosine avec le chlorotriméthylsilane, notamment dans la pyridine, puis on ajoute 1,2 équivalent molaire de chlorure de benzoyle. Après formation du dérivé N-benzoyle correspondant, on ajoute du fluorure de sodium dans un mélange eau/méthanol en milieu acide.

Selon l'étape 2, on ajoute au composé obtenu de l'hydrure de sodium dans le diméthylformamide et on ajoute un tosylate de formule R₃OTs (Ts désigne le groupe tosyle) ou un composé halogéné de formule R₃X (avec X désignant Cl, Br ou I).

Selon l'étape 3, on ajoute une solution aqueuse à 10 % d'acide chlorhydrique et du méthanol et on porte le mélange à reflux pendant 10 minutes.

Selon l'étape 4, on ajoute de l'hydrure de sodium dans le diméthylformamide puis 2 équivalents molaires de composé tosylate de formule R₃OTs (Ts désigne le groupe tosyle) ou de composé halogéné de formule R₃X (avec X désignant Cl, Br ou I).

Selon l'étape 5, on ajoute une quantité catalytique de méthylate de sodium dans du méthanol.

### Deuxième procédé

Les composés de formule (I) pour lesquels R₁ et R₂ forment ensemble un radical ispropylidène et R₃ désigne un radical hydrocarboné tel que défini précédemment , peuvent notamment être préparés selon le schéma II réactionnel suivant :

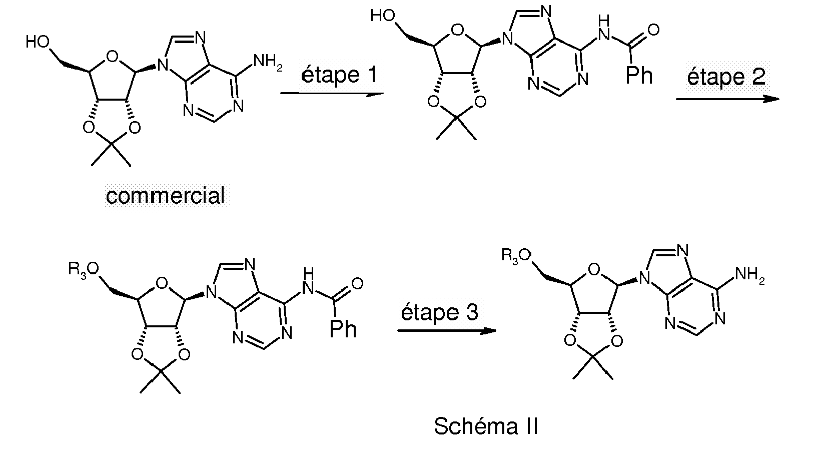

Selon l'étape 1, on fait réagir l'isopropylidène adénosine avec le chlorotriméthylsilane, notamment dans la pyridine, puis on ajoute 1,2 équivalent molaire de chlorure de benzoyle. Après formation du dérivé N-benzoyle correspondant, on ajoute du fluorure de sodium dans un mélange eau/méthanol en milieu acide.

Selon l'étape 2, on ajoute au composé obtenu de l'hydrure de sodium dans le diméthylformamide et on ajoute un tosylate de formule R₃OTs (Ts désigne le groupe tosyle) ou un composé halogéné de formule R₃X (avec X désignant Cl, Br ou I).

Selon l'étape 3, on ajoute une quantité catalytique de méthylate de sodium dans du méthanol.

### Troisième procédé

Les composés de formule (I) pour lesquels R₁ = R₂ et désignent un radical hydrocarboné et R₃ désigne un radical -COR₄ ou un groupe ester issu de la biotine, tels que définis précédemment, peuvent notamment être préparés selon le schéma III réactionnel suivant :

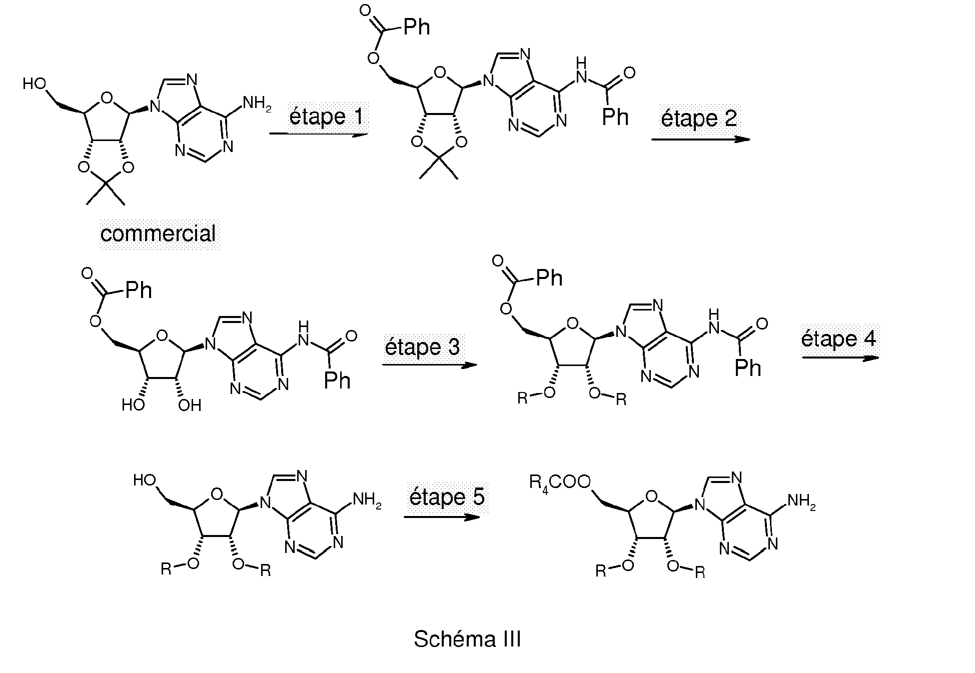

Selon l'étape 1, on fait réagir l'isopropylidène adénosine avec 2,1 équivalents molaires de chlorure de benzoyle , notamment dans la pyridine.

Puis selon l'étape 2, on ajoute une solution aqueuse à 10 % d'acide chlorhydrique et du méthanol et on porte le mélange à reflux pendant 10 minutes.

Selon l'étape 3, on ajoute de l'hydrure de sodium dans le diméthylformamide puis 2 équivalents molaires de composé halogéné de formule R₃X (avec X désignant Cl, Br ou I).

Selon l'étape 4, on ajoute une quantité catalytique de méthylate de sodium dans du méthanol.

Selon l'étape 5, on ajoute un acide organique de formule R₄COOH en présence de carbonyldiimidazole dans le diméthylformamide à une température d'environ 40 °C.

### Quatrième procédé

Les composés de formule (I) pour lesquels R₁ et R₂ forment ensemble un radical ispropylidène et R₃ désigne un radical -COR₄ ou un groupe ester issu de la biotine, tels que définis précédemment, peuvent notamment être préparés selon le schéma IV réactionnel suivant :

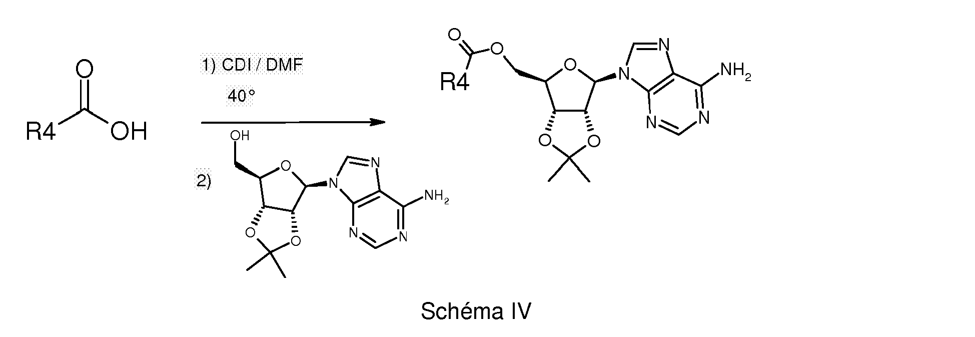

On fait réagir l'acide carboxylique R₄COOH en présence de carbodiimide (CDI) dans le diméthylformamide à une température d'environ 40 °C et on ajoute l'isopropylidène adénosine.

Comme composés de formule (I), on peut citer les composés suivants :
Composé A : 2',3'-isopropylidène-5'-butanoyle-adénosine 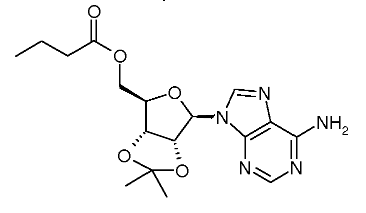
Composé B : 2',3'-isopropylidène-5'-octanoyle-adénosine 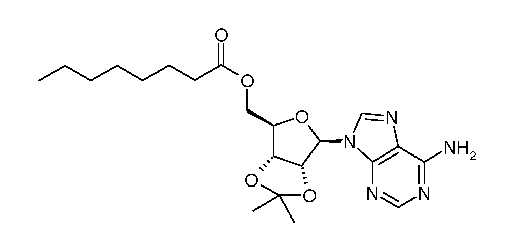
Composé C : 2',3'-isopropylidène-5'-biotinoyle-adénosine 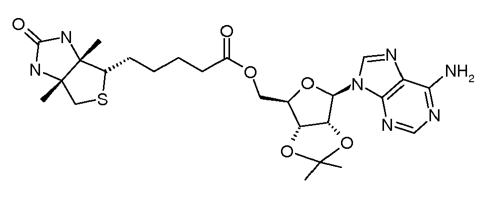
Composé D : 2',3'-isopropylidène-5'-éthyl-adénosine 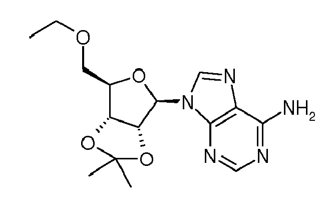
Composé E : 2',3'-isopropylidène-5'-octyle-adénosine 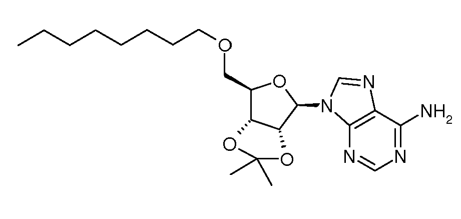
Composé F : 2',3'-diméthyl-5'-éthyl-adénosine 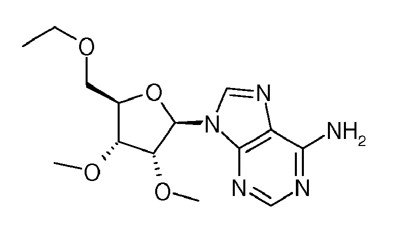
Composé G: 2',3'-diméthyl-5'-butanoyle-adénosine 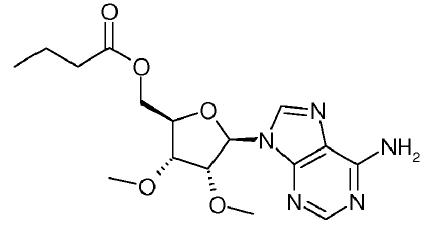
Composé H : 2',3'-isopropylidène-5'-acétyl-adénosine (n ° CAS 15888-38-7)

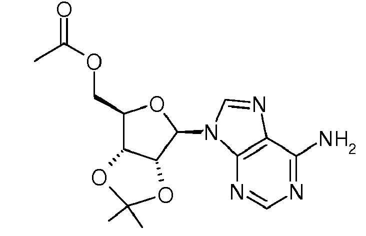

La composition pour l'utilisation selon l'invention est adaptée à une application topique sur la peau. Le milieu physiologiquement acceptable sera préférentiellement un milieu cosmétiquement ou dermatologiquement acceptable, c'est-à-dire sans odeur, couleur ou aspect désagréable, et qui ne génère pas de picotement, tiraillement ou rougeur inacceptable pour l'utilisateur.

Par milieu physiologiquement acceptable, on comprend un milieu compatible avec les matières kératiniques d'êtres humains comme la peau, les muqueuses, les ongles, le cuir chevelu et/ou les cheveux.

La composition pour l'utilisation selon selon l'invention peut être destinée à une application cosmétique ou pharmaceutique, particulièrement dermatologique. De préférence la composition selon l'invention est destinée à une application cosmétique.

La quantité de composés de formule (I) utilisable dans le cadre de l'invention dépend bien évidemment de l'effet recherché.
A titre d'exemple, cette quantité peut aller par exemple de 0,001% à 10% en poids, de préférence de 0,01% à 5% en poids, notamment de 0,1 à 2% en poids, par rapport au poids total de la composition.

La composition peut alors comprendre tous les constituants usuellement employés dans l'application envisagée.

On peut notamment citer l'eau, les solvants, les huiles d'origine minérale, animale et/ou végétale, les cires, les pigments, les charges, les tensioactifs, les actifs cosmétiques ou dermatologiques, les filtres UV, les polymères, les gélifiants, les conservateurs.

Bien entendu l'homme du métier veillera à choisir ce ou ces éventuels composés complémentaires, et/ou leur quantité, de manière telle que les propriétés avantageuses des composés selon l'invention ne soient pas, ou substantiellement pas, altérées par l'adjonction envisagée.

La composition pour l'utilisation selon l'invention peut se présenter sous toutes les formes galéniques normalement utilisées dans les domaines cosmétique et dermatologique; elle peut être notamment sous forme d'une solution aqueuse, hydroalcoolique, éventuellement gélifiée, d'une dispersion du type lotion éventuellement biphasée, d'une émulsion huile-dans-eau ou eau-dans-huile ou multiple, d'un gel aqueux, d'une dispersion d'huile dans une phase aqueuse à l'aide de sphérules, ces sphérules pouvant être des nanoparticules polymériques telles que les nanosphères et les nanocapsules ou, mieux, des vésicules lipidiques de type ionique et/ou non-ionique.

Lorsque la composition pour l'utilisation selon l'invention est une émulsion, la proportion de la phase grasse peut aller de 5 à 80 % en poids, et de préférence de 5 à 50 % en poids par rapport au poids total de la composition. Les huiles, les émulsionnants et les éventuels coémulsionnants utilisés dans la composition sous forme d'émulsion sont choisis parmi ceux classiquement utilisés dans le domaine considéré. L'émulsionnant et le coémulsionnant sont présents, dans la composition, en une proportion pouvant aller de 0,3 à 30 % en poids, et de préférence de 0,5 à 20 % en poids par rapport au poids total de la composition.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sur la peau sous forme d'aérosol. Elle peut également se présenter sous forme solide, et par exemple sous forme de stick.

Cette composition peut constituer une crème de nettoyage, de protection, de traitement ou de soin pour le visage, pour les mains, pour les pieds, pour les grands plis anatomiques ou pour le corps (par exemple crèmes de jour, crèmes de nuit, crèmes démaquillantes, crèmes de fond de teint, crèmes anti-solaires); un fond de teint fluide, un lait de démaquillage, un lait corporel de protection ou de soin, un lait anti-solaire; une lotion, gel ou mousse pour le soin de la peau, comme une lotion de nettoyage.

Dans un aspect avantageux de l'invention, les compositions utilisées peuvent comporter en plus au moins un agent desquamant, et/ou au moins un agent apaisant, et/ou au moins agent photoprotecteur organique et/ou au moins un agent photoprotecteur inorganique.

Par "agent desquamant", on entend tout composé capable d'agir :
- soit directement sur la desquamation en favorisant l'exfoliation, tel que les β-hydroxyacides, en particulier l'acide salicylique et ses dérivés (dont l'acide n-octanoyl 5-salicylique) ; les α-hydroxyacides, tels que les acides glycolique, citrique, lactique, tartrique, malique ou mandélique ; l'urée ; l'acide gentisique ; les oligofucoses ; l'acide cinnamique ; l'extrait de Saphora japonica ; le resvératrol ;
- soit sur les enzymes impliquées dans la desquamation ou la dégradation des cornéodesmosomes, telles que les glycosidases, la stratum corneum chymotryptic enzym (SCCE) voire d'autres protéases (trypsine, chymotrypsine-like). On peut citer les agents chélatant des sels minéraux : l'EDTA ; l'acide N-acyl-N,N',N' éthylène diaminetriacétique ; les composés aminosulfoniques et en particulier l'acide 4-(2-hydroxyéthyl)pipérazine-1-propanesulfonique (HEPES) ; les dérivés de l'acide 2-oxothiazolidine-4-carboxylique (procystéine) ; les dérivés d'acides alpha aminés de type glycine (tels que décrits dans EP-0 852 949, ainsi que le méthyl glycine diacétate de sodium commercialisé par BASF sous la dénomination commerciale TRILON M) ; le miel ; les dérivés de sucre tels que l'O-octanoyl-6-D-maltose et la N-acétyl glucosamine.

Comme agents apaisants utilisables dans la composition selon l'invention, on peut citer : les triterpènes pentacycliques et les extraits de plantes (ex : Glycyrrhiza glabra) en contenant comme l'acide β-glycyrrhétinique et ses sels et/ou ses dérivés (l'acide glycyrrhétinique monoglucuronide, le stearyl glycyrrhetinate, l'acide 3- stéaroyloxy glycyrrhetique), l'acide ursolique et ses sels, l'acide oléanolique et ses sels, l'acide bétulinique et ses sels, un extrait de Paeonia suffruticosa et / ou lactiflora, les sels de l'acide salicylique et en particulier le salicylate de zinc, les phycosaccharides de la société Codif, un extrait de Laminaria saccharina, l'huile de Canola, le bisabolol et les extraits de camomille, l'allantoïne, le Sépivital EPC (diesterphosphorique de vitamine E et C) de Seppic, les huiles insaturées en oméga 3 telles que les huiles de rosier musca, de cassis, d'ecchium, de poisson, des extraits de plancton, la capryloyl glycine, le Seppicalm VG (sodium palmitoylproline et nymphea alba) de Seppic, un extrait du Pygeum, un extrait de Boswellia serrata, un extrait de Centipeda cunnighami, un extrait d'Helianthus annuus, un extrait de Linum usitatissimum, les tocotrienols, les extraits de Cola nitida, le piperonal, un extrait de clou de girofle, un extrait d'Epilobium Angustifolium, l'aloe vera, un extrait de Bacopa moniera, les phytostérols, la cortisone, l'hydrocortisone, l'indométhacine et la beta méthasone.

Les agents photoprotecteurs organiques sont notamment choisis parmi les anthranilates ; les dérivés cinnamiques ; les dérivés de dibenzoylméthane ; les dérivés salicyliques, les dérivés du camphre ; les dérivés de triazine tels que ceux décrits dans les demandes de brevet US 4367390, EP863145, EP517104, EP570838, EP796851, EP775698, EP878469, EP933376, EP507691, EP507692, EP790243, EP944624 ; les dérivés de la benzophénone ; les dérivés de β,β-diphénylacrylate ; les dérivés de benzotriazole ; les dérivés de benzalmalonate ; les dérivés de benzimidazole ; les imidazolines ; les dérivés bis-benzoxazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans s les demandes US5,237,071, US5,166,355, GB2303549, DE 197 26 184 et EP893119 ; et les polymères filtres et silicones filtres tels que ceux décrits notamment dans la demande WO-93/04665 ; les dimères dérivés d'α-alkylstyrène tels que ceux décrits dans la demande de brevet DE 19855649.

Les agents photoprotecteurs inorganiques sont choisis parmi des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP518772 et EP518773.

Les agents photoprotecteurs sont généralement présents dans la composition selon l'invention dans des proportions allant de 0,1 à 20 % en poids par rapport au poids total de la composition, et de préférence allant de 0,2 à 15 % en poids par rapport au poids total de la composition.

Les exemples qui suivent illustrent l'invention sans en limiter la portée. Les composés sont, selon le cas, cités en noms chimiques ou en noms CTFA (International Cosmetic Ingredient Dictionary and Handbook).

### Exemple 1 : Synthèse du 2',3'-isopropylidène-5'-biotinoyle-adénosine (Composé C)

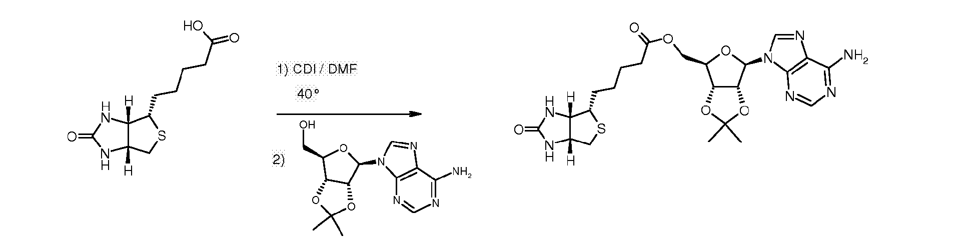

Dans un tricol, sous azote, on a introduit 150 ml de diméthylformamide, 5 g de biotine puis 4 g de carbonyldiimidazole (noté CDI). On a chauffé à 40 °C pendant 1 heure, puis on a additionné 6,3 g de l'isopropylidèneadénosine commercial, puis 5 mg d'amidure de sodium. La solution a été chauffée à 40 °C pendant 24 heures.
On a distillé le diméthylformamide (noté DMF) sans dépasser 40 °C, on a additionné 200 ml de dichlorométhane au résidu.

La phase organique a été lavée 3 fois par 150 ml d'eau, séchée avec du sulfate de sodium et évaporée sous vide à sec.
Le résidu a été purifié sur colonne de silice, éluant dichlorométhane puis dichlorométhane 95 / Méthanol 5 pour conduire à un produit solide, qui a été repris dans du dichlorométhane et précipité par du diéthyle éther.
Le précipité obtenu a été filtré, lavé à l'éther, séché sous vide.

Rendement = 50 %

### Analyses :

RMN DMSO ¹H ¹³C ²D : Spectres conformes

Analyse Elémentaire conforme : C 51.3 ; H 5.89 ; N 18.2 ; O 19.03 ; S 5.92

### Exemple 2 : Synthèse du 2',3'-isopropylidène-5'-butanoyle-adénosine (Composé A)

Ce composé est préparé selon un mode opératoire similaire à celui de l'exemple 1 en utilisant l'acide butyrique à la place de la biotine.

### Exemple 3 : Synthèse du 2',3'-isopropylidène-5'-octanoyle-adénosine (Composé B)

Ce composé est préparé selon un mode opératoire similaire à celui de l'exemple 1 en utilisant l'acide octanoïque à la place de la biotine.

### Exempte 4 : Mise en évidence de l'activité sur la mélanogénèse.

Un test biologique a mis en évidence l'activité dépigmentante des composés de l'exemple 2 (composé A) et du composé H connu.

L'effet modulateur sur la mélanogénèse des composés testé a été mesuré selon la méthode décrite dans le brevet FR-A-2734825 , ainsi que dans l'article de R.Schmidt, P. Krien et M. Régnier, Anal. Bichem., 235(2), 113-18,1996. Ce test est réalisé sur coculture de kératinocytes et de mélanocytes.

Pour le composé testé, il a été déterminé :
- la cytotoxicité, en estimant l'incorporation de la leucine,
- l'activité inhibitrice sur la synthèse de la mélanine, en estimant le rapport de l'incorporation de thiouracile à l'incorporation de leucine, rapporté à 100 % du témoin (le témoin correspond au test réalisé sans composé à testé). Les valeurs des IC50 (concentration pour laquelle 50 % de la synthèse de la mélanine est inhibée) ont été déterminées.

Les résultats sont rassemblés dans le tableau suivant :

| | Cytotoxicité | IC50 (µM) |
|---|---|---|
| Composé ex 1 | Non | 7,4 |
| Composé H | Non | 35 |

Le composé de l'exemple 1 et le composé H sont donc efficaces pour inhiber la mélanogénèse.

On a également effectué le test avec l'adénosine qui n'a pas d'activité dépigmentante significative (à la concentration de 100 µM, seulemnt 20 % de la synthèse de la mélanine est inhibée).

### Exemple 5 :

On prépare une crème blanchissante de soin du visage de type émulsion huile-dans-eau, comprenant (% en poids) :
- composé de l'exemple 2 0,005%
- stéarate de glycérol 2%
- polysorbate 60 (Tween 60 de ICI) 1%
- acide stéarique 1,4%
- triéthanolamine 0,7%
- carbomer 0,4%
- fraction liquide du beurre de karité 12%
- perhydrosqualène 13%
- antioxydant 0,05%
- parfum, conservateur qs
- eau qsp 100%

Une composition similaire est préparée avec le composé de l'exemple 1.

### Exemple 6 :

On prépare un gel dépigmentant pour la peau comprenant (% en poids) :
- Composé H 2%
- hydroxypropylcellulose (Klucel H de Hercules) 1ù
- antioxydant 0;05%
- isopropanol 40%
- parfum, conservateur qs
- eau qsp 100%

Une composition similaire est préparée avec le composé de l'exemple 3.

## Revendications

1. Procédé cosmétique de dépigmentation et/ou de blanchiment de la peau humaine, comprenant l'application sur la peau, d'une composition cosmétique comprenant, dans un milieu physiologiquement acceptable, au moins un composé de formule (I) suivante : 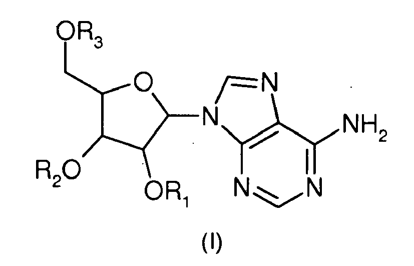 dans laquelle :
- R₁ et R₂ identiques désignent un radical hydrocarboné linéaire saturé en C₁-C₆ ou insaturé en C₂-C₆ , ou ramifié en C₃-C₆ saturé ou insaturé, ou bien encore forment ensemble, avec les atomes d'oxygène auxquels ils sont rattachés un radical isopropylidène ;
- R₃ désigne :
(i) un radical hydrocarboné linéaire saturé en C₁-C₁₀ ou insaturé en C₂-C₁₀, ou ramifié en C₃-C₁₀ saturé ou insaturé, éventuellement substitué par au moins un groupement choisi parmi -OR', -NR'R"; -COOR', -CONR'R", -CF₃, -F , -OCF₃, -CN, -NO₂.
ou (ii) un groupe -COR₄ avec R₄ désignant un radical hydrocarboné linéaire saturé en C₁-C₉ ou insaturé en C₂-C₉ , ou ramifié en C₃-C₉ saturé ou insaturé, éventuellement substitué par au moins un groupement choisi parmi -OR', -NR'R", -COOR', -CONR'R", - CF3, -F , -OCF₃, -CN, -NO₂ ;
ou (iii) un groupe ester issu de la biotine ;
R' et R" désignant un atome d'hydrogène, un radical hydrocarboné linéaire saturé en C₁-C₆ ou insaturé en C₂-C₆ , ou ramifié en C₃-C₆ saturé ou insaturé, éventuellement substitué par au moins un groupement choisi parmi -OZ, -NZZ', -COOZ, Z et Z' désignant, indépendamment l'un de l'autre, un atome d'hydrogène ou un radical hydrocarboné linéaire saturé en C₁-C₆ ou insaturé en C₂-C₆, ou ramifié en C₃-C₆ saturé ou insaturé ;
et ses sels, isomères optiques et solvates.

2. Procédé selon la revendication précédente, **caractérisé par le fait que** le composé (I) est tel que :
- R₁ et R₂ forment ensemble, avec les atomes d'oxygène auxquels ils sont rattachés, un radical isopropylidène ;
- R₃ désigne :
(i) un radical hydrocarboné linéaire saturé en C₁-C₁₀ ou insaturé en C₂-C₁₀, ou ramifié en C₃-C₁₀, saturé ou insaturé ;
ou (ii) un groupe -COR₄ avec R₄ désignant un radical hydrocarboné linéaire en C₁-C₉ ou ramifié en C₃-C₉, saturé ou insaturé ;
ou (iii) un groupe ester issu de la biotine.

3. Procédé selon l'une quelconque des revendications précédentes, **caractérisée par le fait que** le composé (I) est tel que :
- R₁ et R₂ forment ensemble, avec lés atomes d'oxygène auxquels ils sont rattachés, un radical isopropylidène ;
- R₃ désigne :
(i) un radical hydrocarboné linéaire en C₁-C₁₀ ;
ou (ii) un groupe -COR₄ avec R₄ désignant un radical hydrocarboné linéaire saturé en C₁-C₉ ;
ou (iii) un groupe ester issu de la biotine.

4. Procédé selon l'une quelconque des revendications précédentes, **caractérisée par le fait que** le composé (I) est tel que :
R₁ et R₂ forment ensemble, avec les atomes d'oxygène auxquels ils sont rattachés, un radical isopropylidène ;
R₃ désigne :
- un groupe -COR₄ avec R₄ désignant un radical hydrocarboné linéaire en C₁-C₉ ;
- ou un groupe ester issu de la biotine.

5. Procédé selon la revendication 1, **caractérisée par le fait que** le composé (I) est choisi parmi:
- le 2',3'-isopropylidène-5'-butanoyle-adénosine ;
- le 2',3'-isopropylidène-5'-octanoyle-adénosine ;
- le 2',3'-isopropylidène-5'-biotirioyle-adénosine ;
- le 2',3'-isopropylidène-5'-éthyl-adénosine ;
- le 2',3'-isopropylidène-5'-octyle-adénosine ;
- le 2',3'-diméthyl-5'-éthyl-adénosine ;
- le 2',3'-diméthyl-5'-butanoyle-adénosine ;
- le 2',3'-isopropylidène-5'-acétyl-adénosine.

6. Procédé selon l'une quelconque des revendications précédentes, **caractérisée par** la fait que le composé de formule (I) est présent en une quantité allant de 0,001 % à 10% en poids, par rapport au poids total de la composition, de préférence allant de 0,01% à 5% en poids.

7. Utilisation cosmétique d'un composé de formule (I) tel que défini selon l'une quelconque des revendications 1 à 5, comme agent blanchissant et/ou dépigmentant de la peau.

8. Utilisation d'un composé de formule (I) tel que défini selon l'une quelconque des revendications 1 à 5, pour la fabrication d'une composition dermatologique destinée à dépigmenter et/ou blanchir la peau.

## Claims

1. Cosmetic process for depigmenting and/or whitening human skin comprising the application, to the skin, of a cosmetic composition comprising, in a physiologically acceptable medium, at least one compound of following formula (I): 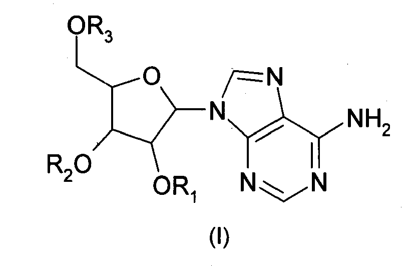 in which:
- R₁ and R₂, which are identical, denote a saturated linear C₁-C₆ or unsaturated linear C₂-C₆ or saturated or unsaturated branched C₃-C₆ hydrocarbon radical or else form, together with the oxygen atoms to which they are attached, an isopropylidene radical;
- R₃ denotes:
(i) a saturated linear C₁-C₁₀ or unsaturated linear C₂-C₁₀ or saturated or unsaturated branched C₃-C₁₀ hydrocarbon radical optionally substituted by at least one group chosen from -OR' , -NR'R'', -COOR', -CONR'R'', -CF₃, -F, -OCF₃, -CN or -NO₂;
or (ii) a -COR₄ group with R₄ denoting a saturated linear C₁-C₉ or unsaturated linear C₂-C₉ or saturated or unsaturated branched C₃-C₉ hydrocarbon radical optionally substituted by at least one group chosen from -OR' , -NR'R'', -COOR' , -CONR'R'', -CF₃, -F, -OCF₃, -CN or -NO₂;
or (iii) an ester group resulting from biotin;
R' and R'' denoting a hydrogen atom or a saturated linear C₁-C₆ or unsaturated linear C₂-C₆ or saturated or unsaturated branched C₃-C₆ hydrocarbon radical optionally substituted by at least one group chosen from -OZ, -NZZ' or -COOZ, Z and Z' denoting, independently of one another, a hydrogen atom or a saturated linear C₁-C₆ or unsaturated linear C₂-C₆ or saturated or unsaturated branched C₃-C₆ hydrocarbon radical;
and its salts, optical isomers and solvates.

2. Process according to the preceding claim, **characterized in that** the compound (I) is such that:
- R₁ and R₂ form, together with the oxygen atoms to which they are attached, an isopropylidene radical;
- R₃ denotes:
(i) a saturated linear C₁-C₁₀ or unsaturated linear C₂-C₁₀ or saturated or unsaturated branched C₃-C₁₀ hydrocarbon radical;
or (ii) a -COR₄ group with R₄ denoting a saturated or unsaturated linear C₁-C₉ or branched C₃-C₉ hydrocarbon radical;
or (iii) an ester group resulting from biotin.

3. Process according to either one of the preceding claims, **characterized in that** the compound (I) is such that:
- R₁ and R₂ form, together with the oxygen atoms to which they are attached, an isopropylidene radical;
- R₃ denotes:
(i) a linear C₁-C₁₀ hydrocarbon radical;
or (ii) a -COR₄ group with R₄ denoting a saturated linear C₁-C₉ hydrocarbon radical;
or (iii) an ester group resulting from biotin.

4. Process according to any one of the preceding claims, **characterized in that** the compound (I) is such that:
- R₁ and R₂ form, together with the oxygen atoms to which they are attached, an isopropylidene radical;
- R₃ denotes:
- a -COR₄ group with R₄ denoting a linear C₁-C₉ hydrocarbon radical;
- or an ester group resulting from biotin.

5. Process according to Claim 1, **characterized in that** the compound (I) is chosen from:
- 2',3'-isopropylidene-5'-butanoyladenosine
- 2',3'-isopropylidene-5'-octanoyladenosine
- 2',3'-isopropylidene-5'-biotinoyladenosine
- 2',3'-isopropylidene-5'-ethyladenosine
- 2',3'-isopropylidene-5'-octyladenosine
- 2',3'-dimethyl-5'-ethyladenosine
- 2',3'-dimethyl-5'-butanoyladenosine
- 2',3'-isopropylidene-5'-acetyladenosine.

6. Process according to any one of the preceding claims, **characterized in that** the compound of formula (I) is present in an amount ranging from 0.001% to 10% by weight, with respect to the total weight of the composition, preferably ranging from 0.01% to 5% by weight.

7. Cosmetic use of a compound of formula (I) as defined according to any one of Claims 1 to 5 as whitening and/or depigmenting agent for the skin.

8. Use of a compound of formula (I) as defined according to any one of Claims 1 to 5 in the manufacture of a dermatological composition intended to depigment and/or whiten the skin.

## Patentansprüche

1. Kosmetisches Verfahren zum Depigmentieren und/oder Bleichen menschlicher Haut, das das Auftragen einer kosmetischen Zusammensetzung auf die Haut umfasst, die in einem physiologisch akzeptablen Medium mindestens eine Verbindung der folgenden Formel (I) enthält: 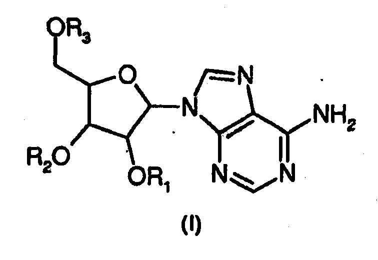 in der Formel:
- R₁ und R₂, die gleich sind, bedeuten eine gesättigte lineare Kohlenwasserstoffgruppe mit 1 bis 6 Kohlenstoffatomen oder eine ungesättigte lineare Kohlenwasserstoffgruppe mit 2 bis 6 Kohlenstoffatomen oder eine gesättigte oder ungesättigte verzweigte Kohlenwasserstoffgruppe mit 3 bis 6 Kohlenstoffatomen oder sie bilden gemeinsam mit den Sauerstoffatomen, an die sie gebunden sind, eine Isopropylidengruppe:
- R₃ bedeutet:
(i) eine gesättigte lineare Kohlenwasserstoffgruppe mit 1 bis 10 Kohlenstoffatomen oder eine ungesättigte lineare Kohlenwasserstoffgruppe mit 2 bis 10 Kohlenstoffatomen oder eine gesättigte oder ungesättigte verzweigte Kohlenwasserstoffgruppe mit 3 bis 10 Kohlenstoffatomen,
wobei die Kohlenwasserstoffgruppen gegebenenfalls mit mindestens einer Gruppe substituiert sind, die unter -OR', - NR'R", -COOR', -CONR'R", -CF₃, -F, -OCF₃, -CN, -NO₂ ausgewählt ist;
oder (ii) eine Gruppe -COR₄, wobei R₄ eine gesättigte lineare Kohlenwasserstoffgruppe mit 1 bis 9 Kohlenstoffatomen oder eine ungesättigte lineare Kohlenwasserstoffgruppe mit 2 bis 9 Kohlenstoffatomen oder eine gesättigte oder ungesättigte verzweigte Kohlenwasserstoffgruppe mit 3 bis 9 Kohlenstoffatomen bedeutet, wobei die Kohlenwasserstoffgruppen gegebenenfalls mit mindestens einer Gruppe substituiert sind, die unter -OR', -NR'R", - COOR', -CONR'R", -CF₃, -F, -OCF₃, -CN, -NO₂ ausgewählt ist;
oder (iii) eine Estergruppe, die von Biotin stammt;
wobei R' und R" ein Wasserstoffatom, eine gesättigte lineare Kohlenwasserstoffgruppe mit 1 bis 6 Kohlenstoffatomen oder eine ungesättigte lineare Kohlenwasserstoffgruppe mit 2 bis 6 Kohlenstoffatomen oder eine gesättigte oder ungesättigte verzweigte Kohlenwasserstoffgruppe mit 3 bis 6 Kohlenstoffatomen bedeuten, wobei die Kohlenwasserstoffgruppen gegebenenfalls mit mindestens einer Gruppe substituiert sind, die unter -OZ, -NZZ', -COOZ ausgewählt ist, wobei Z und Z' unabhängig voneinander ein Wasserstoffatom oder eine gesättigte lineare Kohlenwasserstoffgruppe mit 1 bis 6 Kohlenstoffatomen oder eine ungesättigte lineare Kohlenwasserstoffgruppe mit 2 bis 6 Kohlenstoffatomen oder eine gesättigte oder ungesättigte verzweigte Kohlenwasserstoffgruppe mit 3 bis 6 Kohlenstoffatomen bedeuten;
und ihre Salze, optischen Isomere und Solvate.

2. Verfahren nach dem vorhergehenden Anspruch, **dadurch gekennzeichnet, dass** die Verbindung (I) so vorliegt, dass:
- R₁ und R₂ bilden gemeinsam mit den Sauerstoffatomen, an die sie gebunden sind, eine Isopropylidengruppe:
- R₃ bedeutet:
(i) eine gesättigte lineare Kohlenwasserstoffgruppe mit 1 bis 10 Kohlenstoffatomen oder eine ungesättigte lineare Kohlenwasserstoffgruppe mit 2 bis 10 Kohlenstoffatomen oder eine gesättigte oder ungesättigte verzweigte Kohlenwasserstoffgruppe mit 3 bis 10 Kohlenstoffatomen;
oder (ii) eine Gruppe -COR₄, wobei R₄ eine lineare Kohlenwasserstoffgruppe mit 1 bis 9 Kohlenstoffatomen oder eine gesättigte oder ungesättigte verzweigte Kohlenwasserstoffgruppe mit 3 bis 9 Kohlenstoffatomen bedeutet;
oder (iii) eine Estergruppe, die von Biotin stammt;

3. Verfahren nach einem der vorhergehenden Ansprüche, **dadurch gekennzeichnet, dass** die Verbindung (I) so vorliegt, dass:
- R₁ und R₂ bilden gemeinsam mit den Sauerstoffatomen, an die sie gebunden sind, eine Isopropylidengruppe:
- R₃ bedeutet:
(i) eine lineare Kohlenwasserstoffgruppe mit 1 bis 10 Kohlenstoffatomen;
oder (ii) eine Gruppe -COR₄, wobei R₄ eine gesättigte lineare Kohlenwasserstoffgruppe mit 1 bis 9 Kohlenstoffatomen bedeutet;
oder (iii) eine Estergruppe, die von Biotin stammt;

4. Verfahren nach einem der vorhergehenden Ansprüche, **dadurch gekennzeichnet, dass** die Verbindung (I) so vorliegt, dass:
R₁ und R₂ bilden gemeinsam mit den Sauerstoffatomen, an die sie gebunden sind, eine Isopropylidengruppe:
R₃ bedeutet:
- eine Gruppe -COR₄, wobei R₄ eine lineare Kohlenwasserstoffgruppe mit 1 bis 9 Kohlenstoffatomen bedeutet;
- oder eine Estergruppe, die von Biotin stammt;

5. Verfahren nach Anspruch 1, **dadurch gekennzeichnet, dass** die Verbindung (I) ausgewählt ist unter:
- 2',3'-Isopropyliden-5'-butanoyl-adenosin;
- 2',3'-Isopropyliden-5'-octanoyl-adenosin;
- 2',3'-Isopropyliden-5'-biotinoyl-adenosin;
- 2',3'-Isopropyliden-5'-ethyl-adenosin;
- 2',3'-Isopropyliden-5'-octyl-adenosin;
- 2',3'-Dimethyl-5'-ethyl-adenosin;
- 2',3'-Dimethyl-5'-butanoyl-adenosin;
- 2',3'-Isopropyliden-5'-acetyl-adenosin.

6. Kosmetisches Verfahren nach einem der vorhergehenden Ansprüche, **dadurch gekennzeichnet, dass** die Verbindung der Formel (I) in einer Menge von 0,001 bis 10 Gew.-%, bezogen auf das Gesamtgewicht der Zusammensetzung, und vorzugsweise 0,01 bis 5 Gew.-% vorliegt.

7. Kosmetische Verwendung einer Verbindung der Formel (I) nach einem der Ansprüche 1 bis 5 als Bleichmittel und/oder Depigmentierungsmittel für die Haut.

8. Verwendung einer Verbindung der Formel (I) nach einem der Ansprüche 1 bis 5 für die Herstellung einer dermatologischen Zusammensetzung, die dazu vorgesehen ist, die Haut zu depigmentieren und/oder zu bleichen.
